(19) Europäisches Patentamt
European Patent Office
Office européen des brevets

(11) Numéro de publication: **0 038 735**
**B1**

(12) # FASCICULE DE BREVET EUROPEEN

(45) Date de publication du fascicule du brevet: 30.11.83

(21) Numéro de dépôt: 81400552.6

(22) Date de dépôt: 07.04.81

(51) Int. Cl.³: C 07 B 29/00, C 07 C 17/00, C 07 C 19/08, C 07 C 120/04, C 07 C 121/16, C 07 C 121/20, C 07 C 51/353, C 07 C 53/18, C 07 C 67/333, C 07 C 69/63

(54) **Procédé de préparation de produits ayant un groupement perfluoroalkyle en présence de zinc en milieu acide.**

(30) Priorité: 18.04.80 FR 8008721

(43) Date de publication de la demande: 28.10.81 Bulletin 81/43

(45) Mention de la délivrance du brevet: 30.11.83 Bulletin 83/48

(84) Etats contractants désignés: BE CH DE FR GB LI NL SE

(56) Documents cités:
US - A - 3 557 224
US - A - 4 073 817

THE JOURNAL OF ORGANIC CHEMISTRY, vol. 41, no. 5, 5 mars 1976, PENNSYLVANIA (US) N.O. BRACE et al.: "Evidence for free-radical reductive dehalogenation in reaction of Zinc and acid with 1-perfluoroalkyl-2-iodoalkanes and with 1-Perfluoroalkyl-2-iodoalkenes", pages 766-770

(73) Titulaire: P C U K PRODUITS CHIMIQUES UGINE KUHLMANN, Service Propriété Industrielle Tour Manhattan, F-92087 Paris La Défense 2 Cédex 21 (FR)

(72) Inventeur: Blancou, Hubert Jean, Le Sylvie Bâtiment A Rue Paul Rimbaud, F-34000 Montpellier (FR)
Inventeur: Commeyras, Auguste André Aimé, 6, Impasse des Ecoles, F-34960 Claplers (FR)
Inventeur: Teissedre, Robert, 505 rue des Quatre Vents, F-34100 Montpellier (FR)

(74) Mandataire: Bernard, Nicole et al, PCUK Produits Chimiques Ugine Kuhlmann Service Propriété Industrielle Tour Manhattan - Cedex 21, F-92087 Paris la Défense 2 (FR)

EP 0 038 735 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

Procédé de préparation de produits ayant un groupement perfluoroalkyle en présence de zinc en milieu acide

La présente invention a pour objet un procédé de préparation de produits fluorés de formule:

$$R_F-CHR_1-CHR_2-y \qquad (I)$$

dans laquelle:

- $R_F$ représente une chaîne perfluorée droite ou ramifiée $C_nF_{2n+1}$, n étant un nombre entier de 1 à 20.
- y est un groupement fonctionnel choisi parmi les groupements hydroxy, carboxy, nitrile, ester, halogènes, époxyde, amide, acyloxy, thiol, thioeter ou thiocyanate ou une chaîne hydrocarbonée substituée par un ou plusieurs de cas groupements fonctionnels.
- $R_1$ et $R_2$, identiques ou différents, peuvent être soit y, soit un atome d'hydrogène, soit un radical alkyle, cyclo-alkyle ou aryle, ou également constituer ensemble un radical divalent et former ainsi un dérivé cyclique.

Le procédé de préparation de ces produits est caractérisé par le fait qu'on fait réagir un iodure de perfluoroalkyle $R_FI$ avec une oléfine de formule:

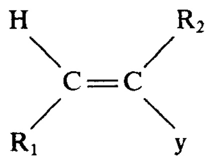

(II)

en présence de poudre de zinc dispersée dans un solvant acide.

Les groupes fonctionnels constituant les substituants de l'oléfine (II) peuvent être très variés et sont par exemple des groupes hydroxy ($-OH$), des atomes d'halogène, des fonctions acide ($-COOH$), nitrile, ester ($-COOR$), époxyde, amide, acyloxy ($-OCOR$), thiol ($-SH$), thioéther ($-SR$) ou thiocyanate ($-SCN$).

Les produits obtenus selon le procédé de l'invention ne sont pas tous des produits nouveaux mais les méthodes de préparation connues à ce jour nécessitent toutes au moins deux étapes réactionnelles. Ainsi les dérivés où $R_1$ et $R_2$ sont de l'hydrogène, c'est-à-dire

$$R_F-CH_2-CH_2-y$$

ont été obtenus par réactions de substitution nucléophile des $R_FCH_2CH_2I$ éventuellement suivies de transformations chimiques appropriées. Le dérivé $R_F-CH_2CH_2COOH$ a par exemple été obtenu par hydrolyse du nitrile $R_FCH_2CH_2-CN$ luimême obtenu par réaction de $R_FC_2H_4I$ avec un cyanure métallique (B.F. 1 560 544), le $R_FC_2H_4I$ étant préparé par addition radicalaire du $R_FI$ sur de l'éthylène.

Les mêmes produits ont aussi été obtenus par addition radicalaire des $R_FI$ sur des oléfines portant des substituants fonctionnels. Cette réaction décrite par exemple dans les brevets américain 3 145 222 et français 2 103 459 fournit des dérivés iodés qui peuvent être transformés, en particulier par réduction avec le zinc ou par hydrogénolyse, en produits identiques à ceux du procédé de l'invention:

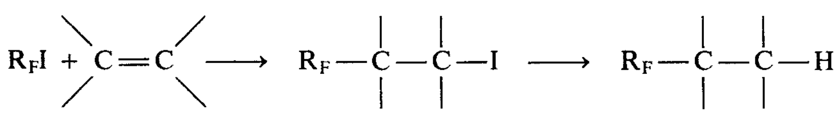

D'après le certificat d'addition français n° 2 373 503 il est possible de faire réagir des iodures de perfluoroalkyle avec des oléfines en milieu diméthylsulfoxyde et en présence de couples métalliques. L'exemple n° 2 de ce certificat d'addition décrit ainsi la réaction de $C_6F_{13}I$ et d'acrylonitrile en milieu DMSO et en présence du couple Zn/Cu qui a fourni $C_6F_{13}C_2H_4CN$ avec un rendement de 50%. Cependant cette réaction ne fournit pas de résultats reproductibles et les rendements restent faibles.

En revanche, il a été constaté que si la réaction est réalisée dans un solvant acide, on obtient de façon reproductible d'excellents résultats selon une méthode très facile à mettre en oeuvre. Le procédé selon l'invention consiste à faire réagir l'iodure de perfluoroalkyle et l'oléfine avec de la poudre de zinc dispersée dans un solvant acide. Ce solvant peut être un acide organique tel que l'acide formique, acétique ou propionique par exemple, un acide minéral (par exemple acide chlorhydrique) ou un mélange d'un acide organique ou minéral et d'un solvant organique tel que le benzène, le toluène ou le dioxanne par exemple. La quantité de solvant utilisé peut être très variable et dépend essentiellement de la nature de l'iodure de perfluoroalkyle et de l'oléfine engagés. On utilise en général environ 100 à 1000 ml de solvant par mole d'iodure de perfluoroalkyle.

Le zinc utilisé dans ce procédé est de la poudre de zinc commerciale mais on a en général intérêt à

activer cette poudre de sinc selon les méthodes connues d'après la littérature (par exemple HOUBEN WEYL 1973 XIII 2 a p. 570 à 574 et 815) soit par attaque avec un acide minéral fort comme par exemple l'acide chlorhydrique ou l'acide sulfurique, soit mieux par formation d'alliages avec d'autres métaux, tels que les couples Zn/Na, Zn/Pb, Zn/Hg ou Zn/Cu. En raison de la facilité de préparation du couple Zn/Cu et de sa très grande réactivité, la plupart des réactions organométalliques du zinc sont faites avec ce couple Zn/Cu et c'est cette méthode d'activation du zinc qui a été utilisée pour l'invention. Le procédé selon l'invention nécessite une quantité stoechiométrique de zinc, mais on peut avantageusement utiliser un léger excès de zinc (5 à 50%) par rapport à l'iodure de perfluoroalkyle.

L'iodure de perfluoroalkyle et l'oléfine sont en général engagés en quantité stoechiométrique, mais en vue de pouvoir transformer quantitativement les réactifs il peut être avantageux d'engager un excès de l'un des deux réactifs. Comptetenu du prix élevé des iodures de perfluoroalkyle, on préfère utiliser dans ce cas un excès de l'oléfine afin d'avoir un taux de transformation total de l'iodure.

Le procédé selon l'invention peut être réalisé dans une grande gamme de température. Pour faciliter la mise en oeuvre du procédé on travaille en général sous la pression atmosphérique et à une température comprise entre la température ambiante et la température d'ébullition du mélange réactionnel, de préférence entre 20 et 100° C.

Le procédé consisté à introduire simultanément, sous agitation, l'iodure de perfluoroalkyle et l'oléfine dans la dispersion du zinc dans le solvant. Selon la nature de l'oléfine, il est aussi possible de faire préalablement un mélange de l'oléfine et de l'iodure de perfluoroalkyle et d'introduire directement ce mélange qui peut d'ailleurs aussi être dilué par le solvant.

Les produits obtenus selon cette méthode sont isolés du milieu réactionnel par des moyens appropriés classiques tels que lavage à l'eau, décantation, extraction, distillation ou filtration.

Les oléfines utilisables dans ce procédé de fabrication de dérivés fluorés sont très variées. On peut citer par exemple l'acrylonitrile, l'acide acrylique et méthacrylique, les esters acryliques et méthacryliques, l'acrylamide, l'alcool allylique, l'acétate de vinyle, l'acétate d'allyle, le chlorure de vinyle, et les dérivés des acides crotonique, maléique, fumarique ou itaconique.

Les produits obtenus selon l'invention peuvent dans certains cas être des mélanges d'isomères, le reste perfluoré pouvant se fixer, selon la nature des restes $R_1$, $R_2$ et y, aussi bien sur l'atome de carbone situé en $\alpha$ qu'en $\beta$ du groupe y. Néanmoins on obtient en général un seul produit bien défini.

En dehors des produits normaux de la réaction, c'est-à-dire les dérivés de formule:

$$R_FCHR_1—CHR_2—y$$

le procédé selon l'invention peut aussi fournir, en quantités plus ou moins importantes, des sous-produits tels que par exemple des produits de couplage comme:

$$\begin{array}{l} R_FCHR_1—CR_2—y \\ \qquad\qquad\ \ | \\ R_FCHR_1—CR_2—y \end{array}$$

On peut par ailleurs aussi obtenir d'autres produits secondaires, particulièrement les dérivés de formule $R_FH$ et $R_F-R_F$. Les proportions de ces différents sous-produits dépendent principalement de la nature des oléfines engagées et des conditions opératoires.

## EXEMPLE 1

### préparation de $C_6F_{13}C_2H_4CN$

Dans un réacteur agité en verre, on introduit 400 g d'acide propionique et 2 g d'acétate de cuivre $(CH_3COO)_2Cu$ puis 68,3 g de poudre de zinc. On coule ensuite sous agitation, en maintenant la température à 35° C, en 2 h 30 un mélange de:

| | | |
|---|---|---|
| $C_6F_{13}I$ | 446 g | (1 mole) |
| $CH_2=CHCN$ | 53 g | (1 mole) |

A la fin de l'addition le mélange est encore maintenu pendant une heure à 35° C, puis lavé à température ambiante avec 2 fois 400 ml d'eau. On obtient ainsi 365 g de phase organique qui fournit par distillation:

a) 69 g de fraction de tête contenant de faibles quantités d'eau, d'acide propionique et d'acrylonitrile, 74% de $C_6F_{13}H$ et 6% de $C_6F_{13}C_2H_4CN$;
b) 253 g de liquide distillant à 79° C sous 5 mm Hg et identifié par spectrométrie RMN, IR et spectrographie de masse et par comparaison avec un échantillon obtenu selon un autre procédé, comme étant $C_6F_{13}C_2H_4CN$ de pureté supérieure à 99,5%;

c) 38 g de résidu contenant 11% de $C_6F_{13}C_2H_4CN$ et des produits lourds constitués principalement par:

- $C_6F_{13}C_2H_4CONH_2$
- 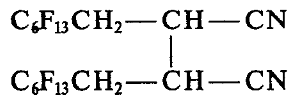
- 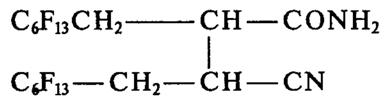
- 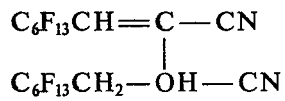

le taux de transformation du $C_6F_{13}I$ est de:

- 16% en $C_6F_{13}H$
- 70% en $C_6F_{13}C_2H_4CN$

## EXEMPLE 2

### préparation de $C_6F_{13}C_2H_4CN$

Un essai réalisé dans les mêmes conditions que l'essai 1, mais en engageant uniquement de la poudre de zinc, en absence de sel de cuivre, a fourni les résultats suivants:

- Taux de transformation en $C_6F_{13}H$ 20%
- Taux de transformation en $C_6F_{13}C_2H_4CN$ 61%

## EXEMPLES 3 à 7

### préparation de $C_6F_{13}C_2H_4CN$

Une série d'essais a été réalisée selon le mode opératoire de l'exemple 1, mais en modifiant la nature du solvant. Ces essais ont été faits en dispersant 4 g de zinc en poudre grossière dans une solution de 100 mg d'acétate de cuivre dans 30 ml d'acide, en faisant varier celui-ci. La dispersion du couple métallique zinc-cuivre est refroidie à température ambiante et on y introduit sous agitation, en une heure, un mélange de 23 g de $C_6F_{13}I$ (0,05 mole) et de 2,7 g d'acrylonitrile. Une heure après la fin de l'addition des deux réactifs, le mélange réactionnel est lavé à l'eau et analysé par résonance magnétique nucléaire. Les résultats sont indiqués dans le tableau I où les pourcentages indiqués sont des pourcentages molaires.

TABLEAU I

| N° essai | Solvant | | $C_6F_{13}I$ non transformé | $C_6F_{13}H$ % | $C_6F_{13}C_2H_4CN$ % |
|---|---|---|---|---|---|
| 3 | $CH_3COOH$ | | <5% | 19 | 81 |
| 4 | $CH_3CH_2COOH$ | | <5% | 20 | 80 |
| 5 | $HCONH_2$ | | <5% | 40 | 60 |
| 6 | $HCOOH$ | 50% | <5% | 46 | 54 |
| | $CH_3COOH$ | 50% | | | |
| 7 | $HCOOH$ | 33% | <5% | 30 | 70 |
| | $CH_3COOH$ | 67% | | | |

## Exemple 8

préparation de $C_6F_{13}C_2H_4CN$

Selon le mode opératoire des exemples 3 à 7, on fait un essai en utilisant comme solvant un mélange de 80 % de benzène et de 20 % d'acide propionique. On obtient dans ce cas un produit contenant en dehors du benzène:

78 % $C_6F_{13}C_2H_4CN$

8 % $C_6F_{13}H$

14 % $C_6F_{13}CH_2-CH-CN$
$\qquad\qquad\qquad\quad |$
$\quad\;\; C_6F_{13}CH_2-CH-CN$

## Exemples 9 à 12

préparation de $C_4F_9-C_2H_4COOH$

Selon le mode opératoire des exemples 3 à 7, on a fait réagir $C_4F_9I$ (17,3 g) et de l'acide acrylique (3,7 g). On a ainsi obtenu l'acide $C_4F_9C_2H_4COOH$ identifié spectroscopiquement et comparé à un échantillon connu de ce produit. Les résultats de cette réaction sont indiqués dans le tableau II pour différents solvants:

TABLEAU II

| N° Exemple | Solvant | % RMN $19_F$ $R_FI$ | $R_FH$ | $R_FC_2H_4COOH$ | Divers |
|---|---|---|---|---|---|
| 9 | $CH_3CH_2COOH$ | <5 | 27 | 73 | |
| 10 | 17% $CH_3CH_2COOH$ 83% benzène | <5 | 6 | 90 | 4 |
| 11 | 83% $CH_3CH_2COOH$ 17% benzène | <5 | 16 | 83 | |
| 12 | 66% $CH_3CH_2COOH$ 33% dioxanne | <5 | 17 | 73 | 10 |

## EXEMPLE 13

Dans les mêmes conditions que celles des exemples 9 à 12, on a fait réagir avec l'acide acrylique le $C_6F_{13}I$ dans un solvant constitué de 17% d'acide propionique et de 83% de benzène. Après traitement on a obtenu un produit contenant d'après l'analyse RMN:

12% $C_6F_{13}H$
71% $C_6F_{13}C_2H_4COOH$
17% dimère non isolé et non identifié

## EXEMPLE 14

Dans les mêmes conditions que celles de l'exemple 13, on a fait réagir $C_8F_{17}I$ avec de l'acide acrylique et on a obtenu:

| | |
|---|---|
| 20% | $C_8F_{17}H$ |
| 40% | $C_8F_{17}C_2H_4COOH$ |
| 40% | produits non identifiés |

## EXEMPLES 15 à 18

### préparation de $C_6F_{13}C_2H_4COOC_2H_5$

En faisant réagir 23 g de $C_6F_{13}I$ et 5 g d'acrylate d'éthyle dans les conditions des exemples 3 à 7, on a obtenu $C_6F_{13}C_2H_4COOC_2H_5$ et un dimère de formule:

$$C_6F_{13}CH_2—CH—COOC_2H_5$$
$$|$$
$$C_6F_{13}CH_2—CH—COOC_2H_5$$

identifiés après séparation par spectrométrie de masse et par RMN. Les proportions relatives des différents produits dépendent en particulier du solvant. Les résultats de ces essais sont indiqués dans le tableau III.

TABLEAU III

| N° Exemple | Solvant | % (RMN) $R_FI$ | $R_FH$ | $R_FC_2H_4COOC_2H_5$ | Dimère |
|---|---|---|---|---|---|
| 15 | $CH_3COOH$ | <3 | 34 | 56 | 8 |
| 16 | $CH_3CH_2COOH$ | <3 | 18 | 71 | 10 |
| 17 | 80% $CH_3CH_2COOH$ 20% benzène | <3 | 12 | 76 | 11 |
| 18 | 20% $CH_3CH_2COOH$ 80% benzène | <3 | 4 | 85 | 11 |

## Exemples 19 à 22

### préparation de $C_4F_9CH_2CH_2OCOCH_3$

Par réaction de $C_4F_9I$ avec de l'acétate de vinyle dans les exemples 3 à 7, on obtient:

$$C_4F_9C_2H_4OCOCH_3$$

et

$$C_4F_9CH_2—CH—OCOCH_3$$
$$|$$
$$C_4F_9CH_2—CH—OCOCH_3$$

qui ont tous deux été isolés et identifiés par spectrométrie de masse et de RMN. Les résultats sont consignés sur le tableau IV, les pourcentages des différents produits ayant été déterminés par chromatographie gazeuse.

TABLEAU IV

| N° Exemple | Solvant | | $R_FH$ % | $R_FC_2H_4OCOCH_3$ % | Dimères % |
|---|---|---|---|---|---|
| 19 | $CH_3COOH$ | | 35 | 35 | 29 |
| 20 | $CH_3CH_2COOH$ | | 13 | 60 | 26 |
| 21 | $CH_3CH_2COOH$ | 20% | 2 | 75 | 21 |
| | benzène | 80% | | | |
| 22 | $CH_3CH_2COOH$ | 66% | 13 | 67 | 18 |
| | dioxanne | 34% | | | |

### Exemple 23

En faisant réagier dans les conditions des exemples 19 à 22, l'acétate de vinyle (0,05 mole) avec 0,5 mole de $C_6F_{13}I$ dans un mélange de solvants constitué de:

33 % acide propionique

67 % dioxanne

on a obtenu après lavage à l'eau un produit contenant d'après l'analyse chromatographique:

11 % $C_6F_{13}H$

70 % $C_6F_{13}C_2H_4OCOCH_3$

15 % 
$$\begin{array}{l} C_6F_{13}CH_2-CH-OCOCH_3 \\ \qquad\qquad\quad | \\ C_6F_{13}CH_2-CH-OCOCH_3 \end{array}$$

**Revendications**

1. Procédé de préparation de produits ayant la formule:

$$R_F-CHR_1-CHR_2-y$$

dans laquelle:

- $R_F$ représente une chaîne perfluorée droite ou ramifiée $C_nF_{2n+1}$, n étant un nombre entier de 1 à 20.
- y est un groupement fonctionnel choisi parmi les groupements hydroxy, carboxy, nitrile, ester, halogènes, époxyde, amide, acyloxy, thiol, thioéther ou thiocyanate ou une chaîne hydrocarbonée substituée par un ou plusieurs de ces groupements fonctionnels.
- $R_1$ et $R_2$, identiques ou différents, peuvent être soit y, soit un atome d'hydrogène, soit un radical alkyle, cycloalkyle ou aryle, ou constituer ensemble un radical divalent formant ainsi un dérivé cyclique,

consistant à faire réagir un iodure de perfluoroalkyle et une olefine $CHR_1=CR_2y$, caractérisé par le fait que la réaction a lieu en présence de poudre de zinc dispersée dans un solvant acide.

2. Procédé selon la revendication 1 où le zinc est activé par un acide minéral fort ou par formation d'alliage avec un autre métal.

3. Procédé selon l'une des revendications 1 ou 2 où le solvant acide est un acide organique mélangé ou non à un solvant organique.

4. Procédé selon l'une des revendications 1 ou 2 où le solvant acide est un acide minéral mélangé ou non avec un solvant organique.

5. Procédé selon l'une des revendications 1 à 4 où la poudre de zinc est introduite avec un excès de 5 à 50% de la stoechiométrie par rapport à l'iodure de perfluoroalkyle.

6. Procédé selon l'une des revendications 1 à 5 où la température de la réaction est comprise entre 20 et 100° C.

## Patentansprüche

1. Verfahren zur Herstellung von Verbindungen der Formel:

$$R_F-CHR_1-CHR_2-y$$

in der,

- $R_F$ eine geradkettige oder verzweigte perfluorierte $C_nF_{2n+1}$-Kette bedeutet, in der n eine ganze Zahl von 1 bis 20 ist,
- y eine funktionelle Gruppe ist, die unter den folgenden Gruppen ausgewählt ist: Hydroxy, Carboxy, Nitril, Ester, Halogenid, Epoxid, Amid, Acyloxy, Thiol, Thioäther oder Thiocyanat oder eine mit einem oder mehreren dieser funktionellen Gruppen substituierte Kohlenwasserstoffkette;
- $R_1$ und $R_2$ gleich oder verschieden entweder y oder ein Wasserstoffatom oder einen Alkyl-, Cycloalkyl- oder Arylrest oder zusammen einen zweiwertigen Rest bedeuten und so eine cyclische Verbindung bilden,

durch Umsetzung eines Perfluoralkyljodids und eines Olefins $CHR_1=CR_2y$, dadurch gekennzeichnet, daß die Umsetzung in Gegenwart von Zinkpulver durchgeführt wird, das in einem sauren Lösungsmittel dispergiert ist.

2. Verfahren nach Anspruch 1 bei dem das Zink durch eine starke Mineralsäure oder durch Legierungsbildung mit einem anderen Metall aktiviert ist.

3. Verfahren nach Anspruch 1 oder 2, bei dem das saure Lösungsmittel eine gegebenenfalls mit einem organischen Lösungsmittel vermischte organische Säure ist.

4. Verfahren nach Anspruch 1 oder 2, bei dem das saure Lösungsmittel eine gegebenenfalls mit einem organischen Lösungsmittel vermischte Mineralsäure ist.

5. Verfahren nach einem der Ansprüche 1 bis 4, bei dem das Zinkpulver mit einem Überschuß von 5 bis 50% gegenüber der Stöchiometrie und bezogen auf das Perfluoralkyljodid zugegeben wird.

6. Verfahren nach einem der Ansprüche 1 bis 5, bei dem die Reaktionstemperatur 20 bis 100°C beträgt.

## Claims

1. Process for the preparation of products having the formula

$$R_F-CHR_1-CHR_2-y$$

in which:

- $R_F$ represents a straight or branched perfluorinated chain $C_nF_{2n+1}$, n being an integer from 1 to 20,
- y is a functional group chosen from amongst the hydroxyl, carboxyl, nitrile, ester, halogen, epoxide, amide, acyloxy, thiol, thioether or thiocyanate groups or a hydrocarbon chain substituted by one or more of these functional groups, and
- $R_1$ and $R_2$, which may be identical or different, can be either y or a hydrogen atom or an alkyl, cycloalkyl or aryl radical or can jointly constitute a divalent radical, thus forming a cyclic derivative,

consisting of reacting a perfluoroalkyl iodide and an olefine $CHR_1=CR_2y$, characterised in that the reaction takes place in the presence of dispersed zinc powder in an acid solvent.

2. Process according to Claim 1, wherein the zinc is activated by means of a strong mineral acid or by formation of an alloy with another metal.

3. Process according to one of Claims 1 or 2, wherein the acid solvent is an organic acid which may or may not be mixed with an organic solvent.

4. Process according to one of Claims 1 or 2, wherein the acid solvent is a mineral acid which may or may not be mixed with an organic solvent.

5. Process according to one of Claims 1 to 4, wherein the zinc powder is introduced in an excess of 5 to 50% of the stoichiometric amount relative to the perfluoroalkyl iodide.

6. Process according to one of Claims 1 to 5, wherein the reaction temperature is between 20 and 100°C.